# FASCICULE DE BREVET EUROPEEN

(11) **EP 0 687 461 B1**
(45) Date de publication et mention de la délivrance du brevet: **09.10.1996**
(21) Numéro de dépôt: 95401081.5
(22) Date de dépôt: 10.05.1995
(51) Int. Cl.: A61K 7/48, A61K 7/42, C09D 133/12, C08L 33/12, A61K 7/032, A61K 7/02, A61K 7/027

(54) **Composition de soin ou de maquillage comprenant une dispersion aqueuse de polymères**
Make-up oder Hautpflegemittel, das eine wässrige Polymer-Dispersion enthält
Make-up or skin care composition comprising an aqueous polymer dispersion

(30) Priorité: 17.06.1994 FR 9407480
(43) Date de publication de la demande: 20.12.1995
(73) Titulaire: L'OREAL, F-75008 Paris (FR)
(72) Inventeur: Lion, Bertrand, F-93190 Livry Gargan (FR); Mondet, Jean, F-93700 Drancy (FR)
(74) Mandataire: Dodin, Catherine

(56) Documents cités:
- EP-A- 0 140 325
- EP-A- 0 353 896
- EP-A- 0 478 284
- GB-A- 2 238 242
- US-A- 4 946 932
- RESEARCH DISCLOSURE, vol.326, 1991, NEW YORK (USA) pages 390 - 391 DISCLOSED ANONYMOUSLY 'Hair grooming compositions'
- WöRTERBUCH DER KOSMETIK. 2. AUFLAGE, 1990, STUTTGART (DE) page 114 H. FEY 'Haarsprays'
- RESEARCH DISCLOSURE, vol.326, 1991, NEW YORK (USA) page 395 DISCLOSED ANONYMOUSLY 'Fast Drying Aqueous Nail Polish'

## Description

La présente demande concerne une composition cosmétique, notamment de soin ou de maquillage, qui comprend au moins un corps gras et au moins dispersion aqueuse de polymère hybride sous forme de particules composites, semblable à un alliage de deux polymères de base susceptible d'être obtenue par une polymérisation radicalaire d'au moins un monomère radicalaire à l'intérieur et/ou partiellement en surface, de particules préexistantes de polymère de type polyester.

L'invention concerne également l'utilisation d'une telle dispersion, notamment en tant qu'agent filmogène, dans une composition cosmétique comprenant au moins un corps gras.

On connaît dans le document RESEARCH DISCLOSURE Vol. 326, 1991, NEW YORK, pages 390-391 des dispersions aqueuses constituées du mélange d'une dispersion aqueuse de polyester ou de polyesteramide à groupements sulfonates et d'au moins une solution de polymère hydrosoluble, comme agent filmogène dans des compositions pour la fixation des cheveux ne contenant pas de corps gras.

On connaît également dans le document RESEARCH DISCLOSURE Vol. 326, 1991, NEW YORK, page 395 des dispersions aqueuses de polymère hybride sous forme de particules composites, semblable à un alliage de deux polymères de base obtenues par polymérisation radicalaire d'un mélange contenant un monomère α, β-insaturé, de l'eau, un amorceur et un polyester ou polyesteramide à groupements sulfonates. Ces dispersions aqueuses de polymère hybride sont utilisées en présence ou non de colorants ou de pigments pour la préparation de vernis à ongles dépourvus de corps gras.

Le document EP-A-0140 325 décrit des compositions cosmétiques contenant comme agent filmogène un copolymère constitué de 80-98% mole de monomère alcool vinylique et de 2-20% mole de monomère alkylvinyléther en C1-C6, linéaire ou ramifié, ledit copolymère étant utilisé sous forme de solution aqueuse.

Il est connu d'utiliser des dispersions aqueuses de polymère en tant qu'agent filmogène, dans des compositions de maquillage comprenant des corps gras, par exemple dans des mascaras. Les propriétés des dispersions aqueuses ainsi obtenues, donc des compositions cosmétiques finales, dépendent de la nature des polymères, et donc des monomères, à partir desquels elles sont préparées. Il peut toutefois être intéressant de pouvoir modifier légèrement ces propriétés, par exemple en accentuant/optimisant une propriété particulièrement intéressante, ou en en développant une nouvelle que ladite dispersion ne pourrait avoir de par sa composition propre.

L'invention a pour but de proposer une composition cosmétique comprenant un corps gras ainsi qu'une dispersion aqueuse d'un polymère choisi dans le groupe constitué par les polyesters, les polyesteramides et les alkydes, ladite dispersion, donc ladite composition, présentant des propriétés améliorées par rapport aux dispersions de polyesters, polyesteramides et/ou alkydes de l'art antérieur.

Un objet de l'invention est donc une composition cosmétique comprenant au moins un corps gras et dispersion aqueuse de polymère hybride sous forme de particules composites, semblable à un alliage de deux polymères de base susceptible d'être obtenue par une polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes.

Un autre objet de l'invention est l'utilisation d'une dispersion aqueuse de polymère hybride sous forme de particules composites, semblable à un alliage de deux polymères de base susceptible d'être obtenue par une polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes, comme agent filmogène dans une telle composition, ou encore afin d'améliorer les propriétés de rémanence à l'eau ou de démaquillage de ladite composition.

En particulier, lorsque la composition se présente sous la forme d'un mascara ou d'un eye liner, l'utilisation d'une dispersion selon l'invention permet d'améliorer les propriétés d'allongement du ci, de rémanence à l'eau et/ou de démaquillage de la composition.

Dans la suite de la présente description, on entend par "polyester", tout polymère, seul ou en mélange, choisi dans le groupe constitué par les polyesters, les polyesteramides et les alkydes.

On a constaté que l'utilisation de dispersions aqueuses selon l'invention, c'est-à-dire de dispersions aqueuses de polymères hybrides de polyesters, permet d'obtenir une composition cosmétique ayant des propriétés, par exemple de rémanence à l'eau, ou bien de démaquillage, ou encore filmogène, qui sont améliorées, propriétés qu'il n'est pas possible d'obtenir en utilisant, par exemple, un simple mélange de dispersions aqueuses préexistantes de polyester et de polymère acrylique et/ou vinylique.
Un avantage de la présente invention est donc de pouvoir, à partir d'une dispersion aqueuse de polymère déjà existante, développer et/ou optimiser certaines propriétés particulièrement intéressantes, de manière relativement contrôlée.
Ainsi, si l'on prend pour exemple un mascara à appliquer sur les cils, on peut, selon la nature des polymères et monomères utilisés pour former la dispersion, obtenir un mascara dont la rémanence à l'eau est améliorée, ou bien, à l'inverse, dont le démaquillage est facilité, ou encore obtenir un mascara présentant une capacité d'allongement du cil remarquable.

Afin de préparer la composition selon l'invention, on prépare tout d'abord une dispersion aqueuse de polyester.
Cette dispersion peut être préparée par l'homme du métier sur base de ses connaissances techniques générales, en particulier de la manière suivante.
Lorsque le polymère de polyester est insoluble dans l'eau, on peut le dissoudre dans un solvant organique faiblement soluble dans l'eau, ajouter de l'eau de manière à former une émulsion, puis évaporer le solvant organique de manière à obtenir une dispersion aqueuse du polymère de polyester dans l'eau présentant un taux de matière sèche d'environ 30-50% en poids.
Lorsque le polymère de polyester est autodispersible dans l'eau, cette étape peut être évitée si le polymère comporte suffisamment de groupes hydrophiles.

La dispersion aqueuse de "polyester" utilisée peut être une dispersion aqueuse de polyester anionique, cationique, non ionique ou amphotère, de polyesteramides, d'alkydes c'est-à-dire de polyesters à chaîne grasse, seul ou en mélange.
La dispersion peut également être une dispersion de polyesters à groupements ionisables latéraux, tels que sulfonique ou carboxylique.
Le polyester peut comporter des groupements insaturés, par exemple lorsqu'il est obtenu par polycondensation d'un diol ou d'une diamine avec un anhydride d'acide insaturé, l'anhydride maléique par exemple. La charge du monomère radicalaire peut dans ce cas réagir ultérieurement avec le polyester insaturé et donner lieu à un greffage et/ou à une réticulation. On obtient ainsi une dispersion d'un polymère hybride greffé et/ou réticulé, qui peut procurer au film obtenu après application de la dispersion, des propriétés mécaniques particulières, telles qu'une amélioration de l'adhérence dudit film.

La dispersion aqueuse de polymères hybrides de polyester selon l'invention est obtenue par polymérisation radicalaire d'au moins un monomère à l'intérieur et/ou partiellement en surface de particules préexistantes de polyester.
Le monomère radicalaire peut être de nature vinylique ou acrylique, et peut être anionique, cationique, non ionique ou amphotère. On peut également utiliser un mélange de monomères de nature différente. Le monomère, ou le mélange de monomères, est, de préférence, insoluble ou faiblement soluble dans l'eau.
Parmi les monomères susceptibles d'être employés, on peut citer les esters d'acide acrylique ou méthacrylique, tels que les acrylate ou méthacrylate de méthyle, d'éthyle, de propyle, de butyle, d'isobutyle, de tertiobutyle, et d'éthyl-2-hexyle; les acrylamides ou méthacrylamides N-substituées ou N,N-substituées; les esters vinyliques tels que l'acétate de vinyle; le styrène.
On peut également utiliser, seul ou en mélange, un monomère vinylique, acrylique ou méthacrylique comportant un ou plusieurs groupes siloxanes, en particulier :
. le monomère de formule CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule CH₂=C(R1)-C(O)-X-(CH₂)ₚ-[Si(CH₃)(R4)-O-]ₙ-Si(CH₃)₂-R3
dans laquelle R1 représente H ou CH₃, X représenfe O ou NH, p est un entier pouvant être nul, R3 et R4 représentent de manière indépendante CH₃ ou un groupe aliphatique, cycloaliphatique ou aromatique, et n est un entier de préférence compris entre 3 et 300.
On peut également utiliser un monomère vinylique, allylique, ester éther ou amide de l'acide acrylique ou métacrylique comportant un ou plusieurs groupes halogénés, en particulier chlorés et/ou fluorés, et/ou comportant un groupe absorbant dans l'UV et pouvant apporter après polymérisation, une certaine photoprotection contre le rayonnement UV, en particulier solaire, par exemple les groupes benzylidène camphre et/ou benzotriazole, substitué ou non, tels que le 2-(2'-hydroxy-5-methacrylyloxyéthyl-phényl)-2-H-benzotriazole.

Lorsque le monomère, ou le mélange de monomères, est sous forme liquide à température ambiante, la polymérisation radicalaire peut être effectuée sans employer de solvant.
Lorsque le monomère, ou le mélange de monomères, est sous forme solide à température ambiante, on peut le dissoudre avant la polymérisation, de préférence dans un solvant organique, par exemple polaire et miscible à l'eau, tel que le méthanol. Dans ce cas, après polymérisation, on peut distiller le solvant organique contenu dans la dispersion aqueuse, si cela est nécessaire.

La préparation des dispersions aqueuse selon l'invention est faite dans des conditions telles que le monomère polymérise à l'intérieur et/ou partiellement en surface des particules du polymère en l'absence de toute nucléation, c'est-à-dire sans qu'il n'y ait de formation de particules nouvelles.
Pour ce faire, on peut introduire le polymère de polyester en dispersion aqueuse présentant un taux de matière sèche de 30-50% en poids dans un réacteur de polymérisation.
On peut alors y ajouter le monomère, ou le mélange de monomère, tel quel ou en solution dans un solvant adéquat, ainsi qu'un amorceur de polymérisation radicalaire.
Selon sa nature, on introduit l'amorceur radicalaire soit sous forme de solution dans un solvant organique, soit sous forme de solution aqueuse, soit encore préalablement dissout dans le mélange de monomères.
Dans le premier cas, il peut être ajouté en même temps que le monomère en solution, et dans le second cas, il peut être ajouté après le monomère.
On peut utiliser un amorceur de polymérisation radicalaire organique, non soluble dans l'eau, de type peroxyde ou percarbonate, tel que le tertiobutylperoxy-2-éthylhexanoate, ou un amorceur organique soluble dans l'eau, ou encore un amorceur minéral tel que le persulfate de potassium.
On prépare donc un mélange aqueux comprenant le polymère de polyester, le monomère et l'amorceur de polymérisation.
On peut également ajouter à ce mélange un stabilisant qui peut être notamment un tensioactif, ou un mélange de tensioactifs, anionique, amphotère, cationique et/ou non ionique. Lorsque le polyester utilisé est lui-même ionique, il est préférable d'utiliser un tensioactif de même caractère ionique ou amphotère.
De préférence, on utilise un tensioactif ionique et polyoxyéthyléné, en une quantité de 0,5-10% en poids de matière sèche par rapport au poids de matière sèche de polyester.
On chauffe ce mélange jusqu'à la température nécessaire de manière à permettre la décomposition de l'amorceur, et l'on poursuit la polymérisation jusqu'à épuisement des monomères.

Les proportions en monomère radicalaire vis-à-vis du polymère de polyester peuvent être de 10-95% en poids de matière sèche de monomère radicalaire et 5-90% en poids de matière sèche de polyester.

On obtient ainsi une dispersion aqueuse de polymère hybride, dont les particules la constituant se présentent sous forme de particules composites, semblables à un "alliage" des deux polymères de base et de taille comparable à celles des particules de polyester avant polymérisation radicalaire.
Les dispersions ainsi obtenues possèdent des propriétés qui leur sont propres, différentes de celles que l'on obtiendrait en mélangeant deux dispersions aqueuses de chacun des constituants.

Les dispersions selon l'invention peuvent être utilisées dans des compositions de soin de la peau, telles que des crèmes, des lotions, des gels ou des solutions, ou dans des compositions de maquillage telles que des rouges à lèvres, des fonds de teint, des fards à joues ou à paupières, des mascaras ou des eye-liners.
On peut également utiliser les dispersions selon l'invention dans des produits destinés à la photoprotection de la peau et/ou des matières kératiniques contre le rayonnement UV, en particulier contre le rayonnement solaire, lorsqu'elles contiennent un monomère adéquat, susceptible d'apporter une certaine protection solaire.
Ces compositions comprennent les ingrédients habituellement utilisés dans le domaine considéré, et peuvent être préparées selon les méthodes usuelles connues de l'homme du métier.
En particulier, ces compositions comprennent au moins un corps gras pouvant être choisis parmi les corps gras usuels tels que les huiles et/ou les cires hydrocarbonées et/ou siliconées, éventuellement volatiles.
Ainsi, on peut employer toute cire connue dans l'art antérieur et notamment les cires minérales telles que les cires microcristallines, la paraffine, le pétrolatum, la vaseline, I'ozokérite, la cire de montan; les cires animales telles que la cire d'abeilles, la lanoline et ses dérivés; les cires végétales telles que les cires de Candellila, d'Ourrury, de Carnauba, du Japon, le beurre de cacao, les cires de fibres de lièges ou de canne à sucre; les huiles hydrogénés, les esters gras et les glycérides concrets à 25°C; les cires synthétiques, telles que les cires de polyéthylène et les cires obtenues par synthèse de Fischer-Tropsch; les cires de silicone.
On peut également employer une huile, ou un mélange d'huiles, usuellement utilisées en cosmétique, parmi lesquelles on peut citer les huiles minérales telles que l'huile de paraffine ou de vaseline; les huiles animales telles que le perhydrosqualène ou l'huile d'arara; les huiles végétales telles que l'huile d'amande douce, de calophyllum, de palme, de ricin, d'avocat, de jojoba, d'olive ou de germes de céréales; les huiles de silicone; des esters d'acide lanolique, d'acide oléique, d'acide laurique, d'acide stéarique ou d'acide myristique par exemple; des alcools tels que l'alcool oléique, l'alcool linoléique ou linolénique, l'alcool isostéarique ou l'octyl dodécanol; des acétylglycérides, des octanoates, décanoates ou ricinoléates d'alcools ou de polyalcools. Ces huiles peuvent également être au moins partiellement volatiles.

La composition peut en outre comprendre des pigments et/ou des nacres et/ou des charges habituellement utilisés en cosmétique.
Les pigments peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique.

Parmi les pigments organiques, on peut citer le noir de carbone, et les laques de baryum, strontium, calcium, aluminium. Parmi les nacres, on peut citer le mica recouvert d'oxyde de titane, d'oxyde de fer, de pigment naturel ou d'oxychlorure de bismuth ainsi que le mica titane coloré. Les charges peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères telles que l'Expancel (Nobel Industrie), le polytrap (Dow Corning) et les microbilles de résine de silicone.
La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingocéryls, des filtres solaires, des tensioactifs, des polymères liposolubles comme les polyalkylènes, notamment le polybutène, les polyacrylates et les polymères siliconés compatibles avec les corps gras. Bien entendu l'homme du métier veillera à choisir les éventuels composés complémentaires, et/ou leur quantité, de manière telles que les propriétés avantageuses de la composition selon l'invention ne soient pas, ou substantiellement pas, altérées par l'adjonction envisagée.

L'invention est illustrée plus en détail dans les exemples suivants, dans lesquels les pourcentages sont donnés en poids.

### Exemple 1

On disperse 50 g de granulés solides de polymère de polyester à groupement sulfonique AQ 38 vendu par Eastman Kodak dans 250 ml d'eau permutée préalablement chauffée à 80°C, tout en maintenant une agitation cisaillante à l'aide d'un disperseur de type Moritz.
On obtient une dispersion ayant une taille moyenne des particules de 40 nm avec une polydispersité de 0,15.
On laisse reposer la dispersion pendant 24 heures puis on l'introduit dans un réacteur préalablement chauffé à 80°C; on ajoute 50 g de méthacrylate de méthyle au goutte à goutte, ce qui prend environ 45 minutes, puis on laisse sous agitation pendant 1 heure à 80°C.
On ajoute 0,5 ml de tertbutylperoxy-2-éthylhexanoate (Trigonox 21S de Akzo) et on laisse réagir 6 heures sous agitation et barbotage d'azote à 80°C.
Le mélange alors obtenu à la même apparence qu'au départ bien que tout le monomère ait polymérisé.
On descend la température du mélange réactionnel à 25°C, on filtre sur toile de Nylon et l'on concentre la dispersion sous pression réduite jusqu'à obtention d'un taux de matière sèche de 40%.
On obtient ainsi une dispersion qui, après une nouvelle filtration, a les caractéristiques suivantes:
. taille moyenne des particules déterminée par un appareil de diffusion quasi-élastique de lumière du type Coulter N4, de Coultronix: 44 nm
. polydispersité: 0,15
   Considérant que la taille des particules dans la dispersion initiale de polyester AQ 38 est de 40 nm (polydispersité : 0,15), on peut donc constater que la polymérisation du monomère n'a presque pas modifié la taille desdites particules initiales.
. absence de double distribution de particules, ce qui signifie que, lors de la polymérisation, on n'a pas créé une seconde population de particules, en plus de la population initiale.

La dispersion obtenue est une dispersion aqueuse d'un polymère hybride dont les particules résultent de la polymérisation radicalaire d'un monomère méthacrylate de méthyle sur et/ou dans les particules d'un polymère préexistant de type polyester à groupement sulfonique.

### Exemples 2 à 5

De manière similaire à celle décrite dans l'exemple 1, on prépare à partir d'une dispersion aqueuse de granulés de polyester sulfonique (AQ 38 de Eastman Kodak), différents polymères hybrides selon le tableau ci-dessous.
L'amorçage est toujours effectué avec 0,5 ml de Trigonox 21S.
Les mesures de taille de particules et de polydispersité sont effectuées pour une dispersion ayant un taux de matière sèche de 40%.

| | polyester | eau ajoutée | monomère | taille des particules | polydispersité |
|---|---|---|---|---|---|
| exemple 2 | 70 g | 250 ml | 30 g de méthacrylate de méthyle | 35 nm | 0,10 |
| exemple 3 | 50 g | 250 ml | 45 g de méthacrylate de méthyle + 5 g de diméthacrylate d'éthylèneglycol | 45 nm | < 0,10 |
| exemple 4 | 70 g | 250 ml | 30 g de méthacrylate d'isobutyle | 45 nm | 0,12 |
| exemple 5 | 70 g | 300 ml | 25 g de méthacrylate de méthyle + 5 g de diméthacrylate d'éthylèneglycol | 32 nm | 0,18 |

On constate, pour tous ces exemples, que l'on obtient une population de particules unique et homogène, dont la taille a été peu modifiée par la polymérisation.

### Exemple 6

On compare les propriétés de filmification des dispersions des polymères selon l'invention, à température ambiante.
On observe que les dispersions des exemples 1, 2, 3 et 5 filmifient lorsqu'on leur ajoute un plastifiant (20 g de monométhyléther de tripropylèneglycol pour 100 g de matière sèche de dispersion) et permettent l'obtention de films homogènes et transparents après séchage.

### Exemple 7

On disperse 40 g de granulés solides de polymère de polyester à groupement sulfonique AQ 38 vendu par Eastman Kodak dans 160 ml d'eau permutée préalablement chauffée à 80°C, tout en maintenant une agitation cisaillante à l'aide d'un disperseur de type Moritz. On laisse reposer la dispersion pendant 24 heures puis on l'introduit dans un réacteur préalablement chauffé à 80°C; on ajoute 15,8 g d'une solution aqueuse d'OCTARON PS20 (nonylphénol polyoxyéthyléné (4,5 OE) sulfaté en bout de chaîne) vendu par Seppic à 20,25% de matière sèche.
Après agitation, on introduit 0,4 ml de tertbutylperoxy-2-éthylhexanoate (Trigonox 21S de Akzo) et on porte, sous agitation et barbotage d'azote, à 80°C. On ajoute 10 g d'acrylate de butyle et on laisse réagir pendant 8 heures.
On descend la température du mélange réactionnel à 25°C, on filtre sur toile de Nylon et l'on concentre la dispersion sous pression réduite jusqu'à obtention d'un taux de matière sèche de 33%.
On obtient ainsi une dispersion qui a les caractéristiques suivantes:
. taille moyenne des particules déterminée par un appareil de diffusion quasi-élastique de lumière du type Coulter N4, de Coultronix: 25 nm
. polydispersité: 0,4

### Exemple 8

On prépare une disperse aqueuse de 2,5 g de polymère de polyester à groupement sulfonique AQ 38 d'Eastman Kodak dans 200 ml d'eau permutée. On l'introduit dans un réacteur préalablement chauffé à 80°C; on ajoute 2,5 g de lauryl sulfate de sodium, puis 0,25 g de persulfate de potassium et 0,25 g d'hydrogénocarbonate de sodium.
On chauffe à 72°C puis on ajoute un mélange de 42,75 g d'acrylate de tertiobutyle et 4,75 g de macromonomère siliconé vendu par 3M (poids moléculaire 9000 à 12 000).
On maintient le tout à 72°C sous agitation pendant 24 heures puis on descend la température du mélange réactionnel à 25°C, on filtre sur toile de Nylon et l'on concentre la dispersion sous pression réduite jusqu'à obtention d'un taux de matière sèche de 20%.
La dispersion obtenue est une dispersion aqueuse d'un polymère hybride dont les particules résultent de la polymérisation radicalaire d'un mélange de macromonomère siliconé + acrylate de tertiobutyle, sur et/ou dans les particules d'un polymère préexistant de type polyester à groupement sulfonique.

### Exemple 9

On prépare une disperse aqueuse de 2,5 g de polymère de polyester à groupement sulfonique AQ 38 d'Eastman Kodak dans 200 ml d'eau permutée. On l'introduit dans un réacteur préalablement chauffé à 80°C; on ajoute 2,5 g de lauryl sulfate de sodium, puis 0,25 g de persulfate de potassium et 0,25 g d'hydrogénocarbonate de sodium.
On chauffe à 72°C puis on ajoute un mélange de 42,75 g d'acrylate de tertiobutyle et 4,75 g d'acrylate de perfluorohexyle (ATOCHEM).
On maintient le tout à 72°C sous agitation pendant 24 heures puis on descend la température du mélange réactionnel à 25°C, on filtre sur toile de Nylon et l'on concentre la dispersion sous pression réduite jusqu'à obtention d'un taux de matière sèche de 20%.
La dispersion obtenue est une dispersion aqueuse d'un polymère hybride dont les particules résultent de la polymérisation radicalaire d'un mélange d'acrylates de perfluorohexyle et de tertiobutyle, sur et/ou dans les particules d'un polymère préexistant de type polyester à groupement sulfonique.

### Exemple 10

On prépare une composition de mascara de la manière suivante.
On mélange 11,5 g de stéarate de triéthanolamine, 7,0 g de cires d'abeilles, 4,1 g de cire de Carnauba et 11,4 g de paraffine. On porte le mélange à 85°C et on y ajoute 5,5 g d'oxyde de fer noir.
On prépare un second mélange comprenant 35 ml d'eau, 4,5 g de gomme arabique et 0,16 g d'hydroxyéthylcellulose, que l'on fait chauffer à 85°C.
On combine les deux mélanges et l'on ajoute 21,3 g d'une des dispersions des exemples 1 à 9, diluée ou concentrée de manière à avoir un taux de matière sèche de 25% en poids.
On obtient un mascara présentant de bonnes caractéristiques cosmétiques à l'application sur les cils, avec un allongement conséquent, et une bonne rémanence à l'eau.

## Revendications

1. Composition cosmétique comprenant au moins un corps gras et une dispersion aqueuse de polymère hybride sous forme de particules composites, semblable à un alliage de deux polymères de base susceptible d'être obtenue par une polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes.

2. Composition selon la revendication 1, dans laquelle le monomère radicalaire est choisi parmi les esters d'acide acrylique ou méthacrylique; les acrylamides ou méthacrylamides N-substituées ou N,N-substituées; les esters vinyliques; le styrène; les monomères vinyliques, acryliques ou méthacryliques comportant un ou plusieurs groupes siloxanes; les monomères vinyliques, allyliques, ester éther ou amide de l'acide acrylique ou métacrylique comportant un ou plusieurs groupes halogénés; les monomères vinyliques, allyliques, ester éther ou amide de l'acide acrylique ou métacrylique comportant un groupe absorbant dans l'UV et pouvant apporter après polymérisation, une certaine photoprotection contre le rayonnement UV.

3. Composition selon la revendication 2, dans laquelle le groupe halogéné est choisi parmi un groupe chloré et/ou un groupe fluoré et/ou le groupe absorbant dans l'UV est choisi parmi les groupes benzylidène camphre et/ou benzotriazole, substitué ou non, et notamment le 2-(2'-hydroxy-5-methacrylyloxyéthyl-phényl)-2-H-benzotriazole.

4. Composition selon l'une des revendications 2 à 3, dans laquelle le groupe siloxane est choisi parmi, seul ou en mélange :
. le monomère de formule CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule CH₂=C(R1)-C(O)-X-(CH₂)ₚ -[Si(CH₃)(R4)-O-]ₙ-Si(CH₃)₂-R3
dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier.

5. Composition selon l'une des revendications précédentes, dans laquelle la dispersion aqueuse de polyester est choisie parmi une dispersion aqueuse de polyester anionique, cationique, non ionique ou amphotère; de polyester à groupements ionisables latéraux, tels que sulfonique et/ou carboxylique; de polyester à groupements insaturés; seul ou en mélange.

6. Composition selon l'une des revendications précédentes, dans laquelle les proportions en monomère radicalaire vis-à-vis du polymère préexistant de polyester sont de 10-95% en poids de matière sèche de monomère radicalaire et 5-90% en poids de matière sèche de polymère préexistant.

7. Composition selon l'une des revendications précédentes, se présentant sous la forme d'une composition de soin de la peau.

8. Composition selon l'une des revendications 1 à 6, se présentant sous la forme d'une composition de maquillage telle qu'un rouge à lèvres, un fond de teint, un fard à joues ou à paupières, un mascara et/ou un eye-liner.

9. Composition selon l'une des revendications précédentes, se présentant sous la forme d'un produit destiné à la photoprotection de la peau et/ou des matières kératiniques contre le rayonnement UV.

10. Utilisation cosmétique d'une dispersion aqueuse de polymère hybride telle que définie dans l'une quelconque des revendications 1 à 6, en tant qu'agent filmogène, dans une composition cosmétique comprenant au moins un corps gras.

11. Utilisation cosmétique d'une dispersion aqueuse de polymère hybride telle que définie dans l'une quelconque des revendications 1 à 6, dans une composition cosmétique comprenant au moins un corps gras, afin d'améliorer les propriétés de rémanence à l'eau ou de démaquillage de ladite composition.

12. Utilisation cosmétique d'une dispersion aqueuse de polymère hybride telle que définie dans l'une quelconque des revendications 1 à 6, dans une composition de mascara et/ou d'eye liner comprenant au moins un corps gras, afin d'en améliorer les propriétés d'allongement du ci, de rémanence à l'eau et/ou de démaquillage.

13. Utilisation selon l'une des revendications 10 à 12, dans laquelle le monomère radicalaire est choisi parmi les esters d'acide acrylique ou méthacrylique; les acrylamides ou méthacrylamides N-substituées ou N,N-substituées; les esters vinyliques; le styrène; les monomères vinyliques, acryliques ou méthacryliques comportant un ou plusieurs groupes siloxanes; les monomères vinyliques, allyliques, ester éther ou amide de l'acide acrylique ou métacrylique comportant un ou plusieurs groupes halogénés; les monomères vinyliques, allyliques, ester éther ou amide de l'acide acrylique ou métacrylique comportant un groupe absorbant dans l'UV et pouvant apporter après polymérisation, une certaine photoprotection contre le rayonnement UV.

14. Utilisation selon la revendication 13, dans laquelle le groupe halogéné est choisi parmi un groupe chloré et/ou un groupe fluoré, et/ou le groupe absorbant dans l'UV est choisi parmi les groupes benzylidène camphre et/ou benzotriazole, substitué ou non, et notamment le 2-(2'-hydroxy-5-methacrylyloxyéthyl-phényl)-2-H-benzotriazole.

15. Utilisation selon l'une des revendications 13 à 14, dans laquelle le groupe siloxane est choisi parmi, seul ou en mélange :
- le monomère de formule CH₂=C(CH₃)-C(O)-O-(CH₂)₃-Si-[O-Si(CH₃)₃]₃
- un macromonomère siliconé à terminaison monofonctionnelle vinylique, allylique, ester éther ou amide de l'acide acrylique ou méthacrylique, de formule CH₂=C(R1)-C(O)-X-(CH₂)ₚ -[Si(CH₃)(R4)-O-]ₙ-Si(CH₃)₂-R3
dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier.

16. Utilisation selon l'une des revendications 10 à 15, dans laquelle la dispersion aqueuse de polyester est choisie parmi une dispersion aqueuse de polyester anionique, cationique, non ionique ou amphotère; de polyester à groupements ionisables latéraux, tels que sulfonique et/ou carboxylique; de polyester à groupements insaturés; seul ou en mélange.

17. Utilisation selon l'une des revendications 10 à 16, dans laquelle les proportions en monomère radicalaire vis-à-vis du polymère préexistant de polyester sont de 10-95% en poids de matière sèche de monomère radicalaire et 5-90% en poids de matière sèche de polymère préexistant.

18. Utilisation selon l'une des revendications 10, 11 et 13 à 17, dans une composition se présentant sous la forme d'une composition de soin de la peau.

19. Utilisation selon l'une des revendications 10 à 17, dans une composition se présentant sous la forme d'une composition de maquillage.

20. Utilisation selon l'une des revendications 10 à 19, dans une composition destinée à la photoprotection de la peau et/ou des matières kératiniques contre le rayonnement UV.

## Claims

1. Cosmetic composition comprising at least one fatty substance and an aqueous dispersion of a hybrid polymer in the form of composite particles similar to an alloy of two basic polymers which can be obtained by radical polymerization of at least one radical monomer within and/or partially at the surface of pre-existing particles of at least one polymer chosen from the group composed of polyesters, polyesteramides and alkyds.

2. Composition according to Claim 1, in which the radical monomer is chosen from acrylic or methacrylic acid esters; N-substituted or N,N-disubstituted acrylamides or methacrylamides; vinyl esters; styrene; vinyl, acrylic or methacrylic monomers containing one or a number of siloxane groups; vinyl, allyl or acrylic or methacrylic acid ester or amide monomers containing one or a number of halogenated groups; or vinyl, allyl or acrylic or methacrylic acid ester or amide monomers containing a group which absorbs in the UV and which can contribute, after polymerization, a degree of photoprotection against UV radiation.

3. Composition according to Claim 2, in which the halogenated group is chosen from a chlorinated group and/or a fluorinated group and/or the group which absorbs in the UV is chosen from optionally substituted benzotriazole and/or benzylidenecamphor groups and in particular 2-(2'-hydroxy-5'-(methacryloyloxyethyl)phenyl)-2H-benzotriazole.

4. Composition according to either of Claims 2 and 3, in which the siloxane group is chosen from, alone or as a mixture:
• the monomer of formula CH₂=C(CH₃)-C(O)-O-(CH₂)₃-Si-[O-Si(CH₃)₃]₃
• a silicone macromonomer with a monofunctional vinyl, allyl or acrylic or methacrylic acid ester or amide ending, of formula CH₂=C(R₁)-C(O)-X-(CH₂)ₚ-[Si(CH₃)(R₄)-O-]ₙ-Si(CH₃)₂-R₃
in which R₁ represents H or CH₃, X represents O or NH, p is an integer which can be zero, R₃ and R₄ independently represent CH₃ or an aliphatic, cycloaliphatic or aromatic group and n is an integer.

5. Composition according to one of the preceding claims, in which the aqueous polyester dispersion is chosen from an aqueous dispersion of an anionic, cationic, non-ionic or amphoteric polyester; of a polyester containing ionizable side groups, such as sulpho and/or carboxyl; or of a polyester containing unsaturated groups; alone or as a mixture.

6. Composition according to one of the preceding claims, in which the proportions of radical monomer with respect to the pre-existing polyester polymer are 10-95 % by weight of radical monomer on a dry basis and 5-90 % by weight of pre-existing polymer on a dry basis.

7. Composition according to one of the preceding claims, which is provided in the form of a skin care composition.

8. Composition according to one of Claims 1 to 6, which is provided in the form of a make-up composition such as a lipstick, a foundation, a blusher, an eye-shadow, a mascara and/or an eyeliner.

9. Composition according to one of the preceding claims, which is provided in the form of a product intended for the photoprotection of the skin and/or of keratinous substances against UV radiation.

10. Cosmetic use of an aqueous dispersion of a hybrid polymer as defined in any one of Claims 1 to 6, as film-forming agent, in a cosmetic composition comprising at least one fatty substance.

11. Cosmetic use of an aqueous dispersion of a hybrid polymer as defined in any one of Claims 1 to 6, in a cosmetic composition comprising at least one fatty substance, in order to improve the water-resistance or make-up removal properties of the said composition.

12. Cosmetic use of an aqueous dispersion of a hybrid polymer as defined in any one of Claims 1 to 6, in a mascara and/or eyeliner composition comprising at least one fatty substance, in order to improve the eyelash elongation, water-resistance and/or make-up removal properties thereof.

13. Use according to one of Claims 10 to 12, in which the radical monomer is chosen from acrylic or methacrylic acid esters; N-substituted or N,N-disubstituted acrylamides or methacrylamides; vinyl esters; styrene; vinyl, acrylic or methacrylic monomers containing one or a number of siloxane groups; vinyl, allyl or acrylic or methacrylic acid ester or amide monomers containing one or a number of halogenated groups; or vinyl, allyl or acrylic or methacrylic acid ester or amide monomers containing a group which absorbs in the UV and which can contribute, after polymerization, a degree of photoprotection against UV radiation.

14. Use according to Claim 13, in which the halogenated group is chosen from a chlorinated group and/or a fluorinated group and/or the group which absorbs in the UV is chosen from optionally substituted benzotriazole and/or benzylidenecamphor groups and in particular 2-(2'-hydroxy-5'-(methacryloyloxyethyl)phenyl)-2H-benzotriazole.

15. Use according to either of Claims 13 and 14, in which the siloxane group is chosen from, alone or as a mixture:
• the monomer of formula CH₂=C(CH₃)-C(O)-O-(CH₂)₃-Si-[O-Si(CH₃)₃]₃
• a silicone macromonomer with a monofunctional vinyl, allyl or acrylic or methacrylic acid ester or amide ending, of formula CH₂=C(R₁)-C(O)-X-(CH₂)ₚ-[Si(CH₃)(R₄)-O-]ₙ-Si(CH₃)₂-R₃
in which R₁ represents H or CH₃, X represents O or NH, p is an integer which can be zero, R₃ and R₄ independently represent CH₃ or an aliphatic, cycloaliphatic or aromatic group and n is an integer.

16. Use according to one of Claims 10 to 15, in which the aqueous polyester dispersion is chosen from an aqueous dispersion of an anionic, cationic, non-ionic or amphoteric polyester; of a polyester containing ionizable side groups, such as sulpho and/or carboxyl; or of a polyester containing unsaturated groups; alone or as a mixture.

17. Use according to one of Claims 10 to 16, in which the proportions of radical monomer with respect to the pre-existing polyester polymer are 10-95 % by weight of radical monomer on a dry basis and 5-90 % by weight of pre-existing polymer on a dry basis.

18. Use according to one of Claims 10, 11 and 13 to 17, in a composition which is provided in the form of a skin care composition.

19. Use according to one of Claims 10 to 17, in a composition which is provided in the form of a make-up composition.

20. Use according to one of Claims 10 to 19, in a composition intended for the photoprotection of the skin and/or of keratinous substances against UV radiation.

## Patentansprüche

1. Kosmetische Zusammensetzung, die mindestens eine Fettsubstanz und eine wäßrige Dispersion eines Hybridpolymers in Form von Compositpartikeln, ähnlich einer Legierung von zwei Basispolymeren, enthält, die bei der radikalischen Polymerisation mindestens eines radikalisch polymerisierbaren Monomers im Inneren und/oder teilweise an der Oberfläche von bereits vorhandenen Partikeln aus mindestens einem Polymer, das unter den Polyestern, Polyesteramiden und Alkydharzen ausgewählt ist, erhalten werden können.

2. Zusammensetzung nach Anspruch 1, worin das Monomer ausgewählt ist unter Acrylsäureestern oder Methacrylsäureestern; N-substituierten oder N,N-substituierten Acrylamiden und Methacrylamiden; Vinylestern; Styrol; Vinyl-, Acryl- oder Methacrylmonomeren, die eine oder mehrere Siloxangruppen enthalten; Vinylmonomeren, Etherestern und Amiden von Acrylsäure oder Methacrylsäure, die eine oder mehrere halogenhaltige Gruppen enthalten, Vinylmonomeren, Allylmonomeren, Etherestern und Amiden von Acrylsäure oder Methacrylsäure, die eine im UV-Bereich absorbierende Gruppe aufweisen, die nach der Polymerisation einen Lichtschutz gegen UV-Strahlung ergibt.

3. Zusammensetzung nach Anspruch 2, worin die halogenhaltige Gruppe unter einer chlorhaltigen und/oder fluorhaltigen Gruppe ausgewählt ist und/oder die im UV-Bereich absorbierende Gruppe unter der ggf. substituierten Benzylidencamphergruppe und/oder der ggf. substituierten Benzotriazolgruppe und insbesondere unter 2-(2'-Hydroxy-5-methacrylyloxyethylphenyl)-2-H-benzotriazol ausgewählt ist.

4. Zusammensetzung nach einem der Ansprüche 2 bis 3, worin die Siloxangruppe, allein oder im Gemisch, ausgewählt ist unter
. dem Monomer der Formel CH₂=C(CH₃)-C(O)-O-(CH₂)₃-Si-[O-Si(CH₃)₃]₃ und
. endständig monofunktionellen siliconhaltigen Macromonomeren mit einer Vinyl- oder Allyl-Endgruppe oder einer Endgruppe eines Etheresters oder Amids der Acrylsäure oder Methacrylsäure der Formel CH₂=C(R1)-C(O)-X-(CH₂)ₚ-[Si(CH₃)(R4)-O-]ₙ-Si(CH₃)₂-R3, worin bedeuten:
. R1 H oder CH₃,
. X Sauerstoff oder -NH-,
. p eine ganze Zahl oder Null,
. R3 und R4 unabhängig voneinander CH₃ oder eine alipha-tische, cycloaliphatische oder aromatische Gruppe, und
. n eine ganze Zahl.

5. Zusammensetzung nach einem der vorhergehenden Ansprüche, wobei die wäßrige Polyesterdispersion ausgewählt ist unter wäßrigen Dispersionen von anionischen, kationischen, nichtionischen oder amphoteren Polyestern, wäßrigen Dispersionen von Polyestern mit seitlich vorhandenen ionisierbaren Gruppen, wie z.B. Sulfo- oder Carboxygruppen, und wäßrigen Dispersionen von Polyestern mit ungesättigten Gruppen, die allein oder im Gemisch verwendbar sind.

6. Zusammensetzung nach einem der vorhergehenden Ansprüche, worin die Mengenanteile des radikalischen Monomers und des Polyesterpolymers 10 bis 95 Gew.-% Trockensubstanz des radikalischen Monomers und 5 bis 90 Gew.-% Trockensubstanz des Polyesters betragen.

7. Zusammensetzung nach einem der vorhergehenden Ansprüche, die in Form einer Zusammensetzung zur Pflege der Haut vorliegt.

8. Zusammensetzung nach einem der Ansprüche 1 bis 6, die in Form einer Zusammensetzung zum Schminken, wie als Lippenstift, Make-up, Wangenrouge oder Lidschatten, Mascara und/oder Eyeliner, vorliegt.

9. Zusammensetzung nach einem der vorhergehenden Ansprüche, die in Form eines Produkts vorliegt, das zum Lichtschutz der Haut und/oder von Keratinmaterial gegen UV-Strahlung bestimmt ist.

10. Kosmetische Verwendung einer wäßrigen Dispersion eines Hybridpolymers nach einem der Ansprüche 1 bis 6 als Filmbildner in einer kosmetischen Zusammensetzung, die mindestens eine Fettsubstanz enthält.

11. Kosmetische Verwendung einer wäßrigen Dispersion eines Hybridpolymers nach einem der Ansprüche 1 bis 6 in einer Zusammensetzung, die mindestens eine Fettsubstanz enthält, um die Eigenschaften der Wasserfestigkeit und des Anschminkverhaltens der Zusammensetzung zu verbessern.

12. Kosmetische Verwendung einer wäßrigen Dispersion eines Hybridpolymers nach einem der Ansprüche 1 bis 6 in einer Mascara- und/oder Eyeliner-Zusammensetzung, die mindestens eine Fettsubstanz enthält, um die Eigenschaften der Verlängerung der Wimpern, der Wasserfestigkeit und/oder des Abschminkverhaltens zu verbessern.

13. Verwendung nach einem der Ansprüche 10 bis 12, worin das radikalische Monomer ausgewählt ist unter Acrylsäureestern oder Methacrylsäureestern; N-substituierten oder N,N-substituierten Acrylamiden und Methacrylamiden; Vinylestern; Styrol; Vinyl-, Acryl- oder Methacrylmonomeren, die eine oder mehrere Siloxangruppen enthalten; Vinylmonomeren, Etherestern und Amiden von Acrylsäure oder Methacrylsäure, die eine oder mehrere halogenhaltige Gruppen enthalten, Vinylmonomeren, Allylmonomeren, Etherestern und Amiden von Acrylsäure oder Methacrylsäure, die eine im UV-Bereich absorbierende Gruppe aufweisen, die nach der Polymerisation einen Lichtschutz gegen UV-Strahlung ergibt.

14. Verwendung nach Anspruch 13, worin die halogenhaltige Gruppe unter einer chlorhaltigen und/oder fluorhaltigen Gruppe ausgewählt ist und/oder die im UV-Bereich absorbierende Gruppe unter der ggf. substituierten Benzylidencamphergruppe und/oder der ggf. substituierten Benzotriazolgruppe und insbesondere unter 2-(2'-Hydroxy-5-methacrylyloxyethylphenyl)-2-H-benzotriazol ausgewählt ist.

15. Verwendung nach einem der Ansprüche 13 bis 14, worin die Siloxangruppe, allein oder im Gemisch, ausgewählt ist unter:
. dem Monomer der Formel CH₂=C(CH₃)-C(O)-O-(CH₂)₃-Si-[O-Si(CH₃)₃]₃ und
. endständig monofunktionellen siliconhaltigen Macromonomeren mit einer Vinyl- oder Allyl-Endgruppe oder einer Endgruppe eines Etheresters oder Amids der Acrylsäure oder Methacrylsäure der Formel CH₂=C(R1)-C(O)-X-(CH₂)ₚ-[Si(CH₃)(R4)-O-]ₙ-Si(CH₃)₂-R3, worin bedeuten:
. R1 H oder CH₃,
. X Sauerstoff oder -NH-,
. p eine ganze Zahl oder Null,
. R3 und R4 unabhängig voneinander CH₃ oder eine alipha-tische, cycloaliphatische oder aromatische Gruppe, und
. n eine ganze Zahl.

16. Verwendung nach einem der Ansprüche 10 bis 15, wobei die wäßrige Polyesterdispersion ausgewählt ist unter wäßrien Dispersionen von anionischen, kationischen, nichtionischen oder amphoteren Polyestern, wäßrigen Dispersionen von Polyestern mit seitlich vorhandenen ionisierbaren Gruppen, wie z.B. Sulfo- oder Carboxygruppen, und wäßrigen Dispersionen von Polyestern mit ungesättigten Gruppen, die allein oder im Gemisch verwendbar sind.

17. Verwendung nach einem der Ansprüche 10 bis 16, wobei die Mengenanteile des radikalischen Monomers und des Polyesterpolymers 10 bis 95 Gew.-% Trockensubstanz des radikalischen Monomers und 5 bis 90 Gew.-% Trockensubstanz des Polyesters betragen.

18. Verwendung nach einem der Ansprüche 10, 11 und 13 bis 17 in einer Zusammensetzung, die in Form einer Zusammensetzung zur Pflege der Haut vorliegt.

19. Verwendung nach einem der Ansprüche 10 bis 17, in einer Zusammensetzung, die in Form einer Zusammensetzung zum Schminken vorliegt.

20. Verwendung nach einem der Ansprüche 10 bis 19, in einer Zusammensetzung, die zum Lichtschutz der Haut und/oder von Keratinmaterial gegen UV-Strahlung bestimmt ist.
